# EUROPÄISCHE PATENTSCHRIFT

(11) **EP 1 127 879 B1**
(45) Veröffentlichungstag und Bekanntmachung des Hinweises auf die Patenterteilung: **25.05.2005**
(21) Anmeldenummer: 01103732.2
(22) Anmeldetag: 15.02.2001
(51) Int. Cl.: C07D 233/32, C07D 491/04

(54) **Herstellung der 2-Oxo-1,3-dibenzyl-cis-4,5-imidazolidindicarbonsäure bzw. ihres Anhydrids**
Preparation of 2-oxo-1,3-dibenzyl-cis-4,5-imidazolidin dicarboxylic acid and its anhydrid
Procédé pour la préparation de l'acide 1,3-dibenzyle-imidazolidine-2-one- cis-4,5 dicarboxylique et son anhydride

(30) Priorität: 25.02.2000 CH 374002000
(43) Veröffentlichungstag der Anmeldung: 29.08.2001
(73) Patentinhaber: DSM IP Assets B.V., 6411 TE Heerlen (NL)
(72) Erfinder: Fleckenstein, Juergen, 79664 Wehr (DE); Kraemer, Bernd, 79650 Schopfheim (DE); Veits, Joachim, Florence, South Carolina 29501 (US)
(74) Vertreter: Müller, Ingrid, Dr.

(56) Entgegenhaltungen:
- EP-A- 1 081 139
- JP-A- 51 008 270
- US-A- 5 151 525
- P.J.DE CLERCQ ET AL: "Biotin: A Timeless Challenge for Total Synthesis" CHEM.REV., Bd. 97, 1997, Seiten 1755-1792, XP000999266

## Beschreibung

Die vorliegende Erfindung betrifft ein Verfahren zur Herstellung einer Cyclosäure, nämlich von 2-Oxo-1,3-dibenzyl-cis-4,5-imidazolidindicarbonsäure bzw. des entsprechenden Anhydrids, die wichtige Zwischenprodukte in den beispielsweise im Uebersichtsartikel von Pierre J. de Clercq in Chem. Rev. 97, 1755-1792 (1997) beschriebenen mehrstufigen Verfahren zur Herstellung von Biotin (Vitamin H) darstellen.

Die Herstellung der oben erwähnten Cyclosäure ausgehend von meso-2,3-Bis (benzylamino)bernsteinsäure in Form ihres Dialkalimetallsalzes ist bekannt. So beschreibt beispielsweise die US-Patentschrift Nr. 5.151.525 aus dem Jahre 1992 ein derartiges Verfahren, in welchem Phosgen als Reagens zur Verknüpfung der beiden sekundären Stickstoffatome über eine Carbonylgruppe, und daher zur Ringbildung, in einem alkalischen wässrig/organischen Zweiphasen-Lösungsmittelsystem verwendet wird. Dabei wird als im wesentlichen nicht-wassermischbares Lösungsmittel für die Umsetzung Anisol eingesetzt.

Bekanntlich ist das Reagens Phosgen jedoch hochtoxisch und zudem unter Einwirkung anderer Gase oder gewisser Reaktionsflüssigkeiten potentiell explosiv, so dass dessen Verwendung bei unsorgfältiger Handhabung bzw. Ueberwachung äusserst gefährlich ist, und besondere Massnahmen bei dessen Transport, Lagerung und Verwendung, z.B. apparative Sicherheitsvorrichtungen, erforderlich sind.

In der wesentlich älteren Japanischen Patentpublikation (Kokai) Nr. 8270/1976 wird bereits ein Verfahren zur Herstellung der erwähnten Cyclosäure beschrieben, in welchem ebenfalls von meso-2,3-Bis(benzylamino)bernsteinsäure in Form ihres Dialkalimetallsalzes ausgegangen, jedoch anstelle von Phosgen ein Alkyl-, Halogenalkyl- oder Arylchlorformiat als Reagens zur Ringbildung eingesetzt wird. Damit können zwar die oben geschilderten Nachteile von Phosgen vermieden werden, doch hat dieses Verfahren andere ebenfalls gewichtige Nachteile, was der Grund dafür sein dürfte, dass sich bisher der Einsatz von Chlorformiaten nicht durchgesetzt hat. So liess sich die Cyclosäure nur mit dem gemäss den Beispielen bevorzugtesten Reagens, dem Phenylchlorformiat, in erwünschter Ausbeute herstellen. Der Einsatz von Phenylchlorformiat hat allerdings neben den hohen Kosten weitere Nachteile. So fallen einerseits Diphenylcarbonat und Phenol als unvermeidliche Nebenprodukte an, andererseits fällt das Diphenylcarbonat als zähe Masse aus der wässrigen Reaktionslösung aus und verklebt die Reaktoreinbauten sowie die pH-Sonde. Dadurch kann der pH-Wert nicht genau nachgeführt werden, was für die kontrollierte Reaktionsführung von entscheidender Bedeutung ist.

Das Phenylchlorformiat bildet in einem ersten Reaktionsschritt mit dem Dialkalimetallsalz der meso-2,3-Bis(benzylamino)bernsteinsäure unter Abspaltung von Salzsäure eine Monourethan-Zwischenstufe, die dann zur Cyclosäure weiterreagiert. Das entstehende Alkaliphenolat reagiert seinerseits mit Phenylchlorformiat zum Nebenprodukt Diphenylcarbonat. Die entstehende Salzsäure kann durch Zugabe von Alkalilauge neutralisiert werden, wobei der pH-Wert weder zu niedrig, noch zu hoch werden darf. Ist der pH-Wert zu tief, fällt die meso-2,3-Bis(benzylamino)bernsteinsäure aus und kann nicht umgesetzt werden. Zudem verbindet sich die meso-2,3-Bis(benzylamino)bernsteinsäure mit dem ausgefallenen Diphenylcarbonat zu einer zähen Masse, was zu einer weiteren Verklebung der Reaktoreinbauten samt Rührer führt. Bei zu hohen pH-Werten hydrolysiert das Phenylchlorformiat zu schnell, was zu unnötigem Verbrauch führt.

Daneben ist auch die Ausfällung der Cyclosäure durch Zugabe einer starken Säure schwierig zu bewerkstelligen, da diese oft als klebrige Masse anfällt, was im Einklang mit den Angaben in der oben genannten Japanischen Patentpublikation steht und eine weitere Verklebung der Reaktoreinbauten samt Rührer zur Folge haben kann.

Der vorliegenden Erfindung liegt daher die Aufgabe zugrunde, ein Verfahren zur Herstellung von 2-Oxo-1,3-dibenzyl-cis-4,5-imidazolidindicarbonsäure zur Verfügung zu stellen, welches ebenfalls von meso-2,3-Bis(benzylamino)bernsteinsäure in Form ihres Dialkalimetallsalzes ausgeht, wobei einerseits der Einsatz von Phosgen als Reagens zur oben näher erläuterten Ringbildung und andererseits die erheblichen Nachteile der Verfahrensführung gemäss der oben genannten Japanischen Patentpublikation vermieden werden können.

Diese Aufgabe wird überraschend einfach und gut gelöst, indem man die Umsetzung von meso-2,3-Bis(benzylamino)bernsteinsäure in Form ihres Dialkalimetallsalzes mit Phenylchlorformiat in einem Gemisch aus einem mit Wasser mischbaren Ether und Wasser unter alkalischen Bedingungen durchführt, wodurch es möglich wird, das bei der Umsetzung gebildete Diphenylcarbonat in Lösung zu halten. Somit kann man in einem einphasigen Lösungsmittelsystem arbeiten, welches frei von festen Nebenprodukten ist, wodurch wiederum ein Verkleben der Reaktoreinbauten samt Rührer vermieden werden kann.

Bei dem erfindungsgemässen Verfahren handelt es sich um ein Verfahren zur Herstellung von 2-Oxo-1,3-dibenzyl-cis-4,5-imidazolidindicarbonsäure bzw. 2-Oxo-1,3-dibenzyl-4,5-imidazolidindicarbonsäureanhydrid ausgehend von einem Dialkalimetallsalz der meso-2,3-Bis(benzylamino)bernsteinsäure, dadurch gekennzeichnet, dass man
das Dialkalimetallsalz der meso-2,3-Bis(benzylamino)bernsteinsäure mit Phenychlorformiat in einem aus einem etwa 2:1- bis 1:1―Gemisch von einem mit Wasser mischbaren Ether und wässriger Alkalilauge bestehenden Einphasen-Lösungsmittelsystem bei einer etwa 40°C nicht übersteigenden Temperatur umsetzt,
das daraus resultierende einphasige Gemisch, welches als Produkt das Dialkalimetallsalz der 2-Oxo-1,3-dibenzyl-cis-4,5-imidazolidindicarbonsäure enthält, durch Zugabe von Säure auf leicht alkalisch, insbesondere auf pH etwa 7-8, einstellt und mit einem aromatischen Kohlenwasserstoff extrahiert,
die resultierende wässrige Phase, in welcher sich das Produkt noch als Dialkalimetallsalz befindet, abtrennt,
der getrennten wässrigen Phase Tetrahydrofuran zusetzt,
das resultierende wässrig-organische Gemisch auf einen pH-Wert von < 3 (wässrige Phase) ansäuert, um das Dialkalimetallsalz in die freie Säure 2-Oxo-1,3-dibenzyl-cis-4,5-imidazolidindicarbonsäure überzuführen und letztere in die organische Phase aufzunehmen,
die organische Phase, welche die freie Säure enthält, von der wässrigen Phase abtrennt,
der organischen Phase einen aromatischen Kohlenwasserstoff als Lösungsmittel zugibt,
die so erhaltene organische Phase einer Destillation unter vermindertem Druck unterwirft,
um hauptsächlich das Tetrahydrofuran, wenig aromatisches Lösungsmittel sowie verbleibendes Wasser zu entfernen,
und entweder aus der resultierenden übersättigten Lösung des gewünschten Produktes in wasserfreiem aromatischem Lösungsmittel die 2-Oxo-1,3-dibenzyl-cis-4,5-imidazolidindicarbonsäure fällt und diese isoliert
oder zu der resultierenden übersättigten Lösung zusätzlichen aromatischen Kohlenwasserstoff als organisches Lösungsmittel sowie Acetanhydrid zugibt und das Gemisch erhitzt, um die 2-Oxo-1,3-dibenzyl-cis-4,5-imidazolidindicarbonsäure in das alternativ gewünschte 2-Oxo-1,3-dibenzyl-4,5-imidazolidindicarbonsäureanhydrid überzuführen, und dieses isoliert.

Beispiele von mit Wasser mischbaren Ethern sind Tetrahydrofuran und Ethylenglycolether, z.B. Glyme und Diglyme, vorzugsweise Tetrahydrofuran.

Das nachfolgende Reaktionsschema stellt das erfindungsgemässe Verfahren formelmässig dar:

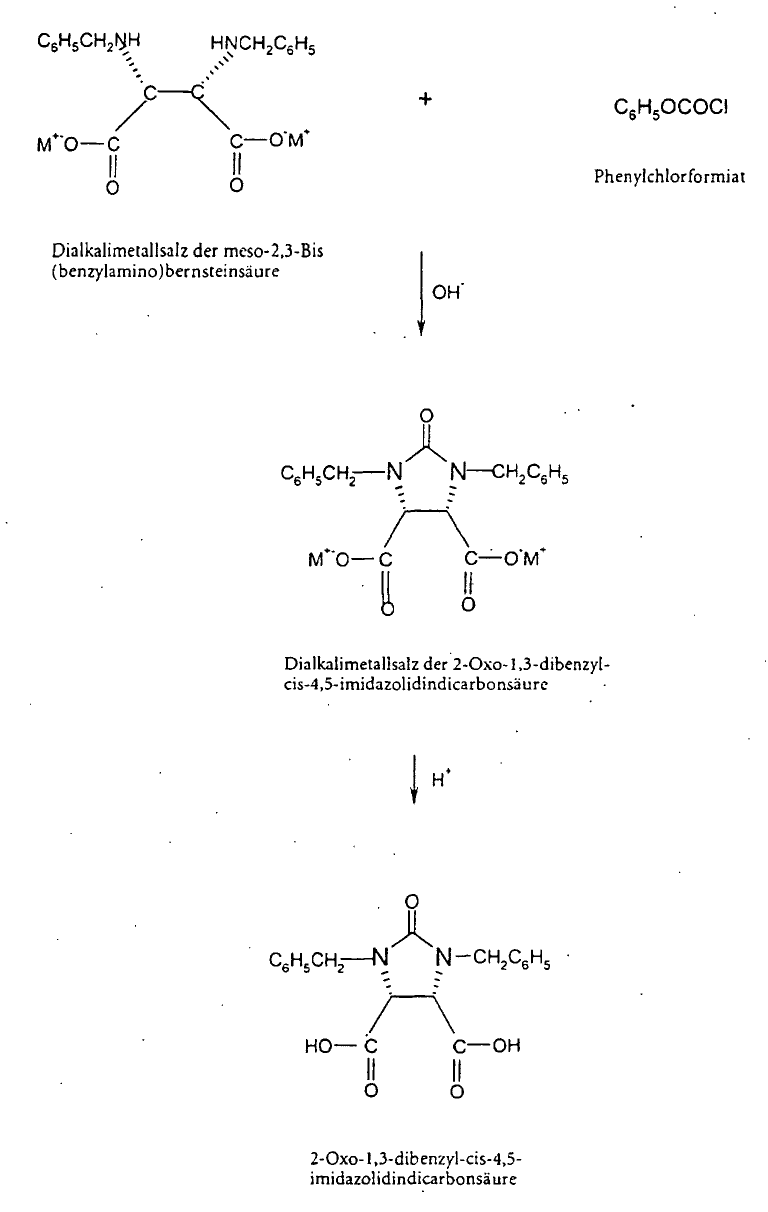

Im obigen Reaktionsschema ist das Alkalimetallion M⁺ zweckmässigerweise das Lithium-, Natrium- oder Kaliumion, vorzugsweise das Kaliumion, so dass als Ausgangsmaterial des erfindungsgemässen Verfahrens zweckmässigerweise das Dilithium-, Dinatrium- oder Dikaliumsalz, vorzugsweise das letztgenannte, verwendet wird.

Zur Zubereitung des Dialkalimetallsalzes der meso-2,3-Bis(benzylamino)bernsteinsäure wird zweckmässigerweise die Säure selber in Wasser, vorzugsweise entionisiertem Wasser, aufgeschlämmt und die resultierende Aufschlämmung mit Alkalilauge, d.h. zweckmässigerweise Lithium-, Natron- oder Kalilauge, vorzugsweise Kalilauge, versetzt, und zwar im allgemeinen bei einem pH-Wert von etwa 9 bis etwa 14, vorzugsweise bei einem pH-Wert von etwa 12 bis etwa 13. Dies ergibt eine alkalische wässrige Lösung des erwünschten Dialkalimetallsalzes. Die Konzentration der zugegebenen Alkalilauge ist nicht kritisch, beträgt allerdings etwa 40 - 50 Gewichtsprozent im Falle der Verwendung einer handelsüblichen Alkalilauge, z.B. Kalilauge. Es werden geeignete Mengen an Wasser und Alkalilauge verwendet, um zweckmässigerweise zu erreichen, dass die Konzentration des gebildeten meso-2,3-Bis(benzylamino)bernsteinsäure-Dialkalimetallsalzes etwa 5 bis etwa 20 Gewichtsprozent, vorzugsweise etwa 10 bis etwa 15 Gewichtsprozent, bezogen auf das Gesamtgewicht der entstehenden klaren alkalischen wässrigen Lösung bei pH etwa 9 bis etwa 14, beträgt. Der mit Wasser mischbare Ether kann gegebenenfalls bereits bei der Aufschlämmung der meso-2,3-Bis(benzylamino)bernsteinsäure dabei sein, so dass die Säure effektiv im wässrigen Ether aufgeschlämmt wird, oder erst nach Zubereiten der wässrigen Dialkalimetallsalzlösung zugegeben werden, um schliesslich die im erfindungsgemässen Verfahren benötigte alkalisch-wässrige Ether-Lösung des Dialkalimetallsalzes zuzubereiten.

Das Zusammenbringen der alkalisch-wässrigen Ether-Lösung des meso-2,3-Bis(benzylamino)bernsteinsäure-Dialkalimetallsalzes mit dem Phenylchlorformiat kann zwar in beliebiger Reihenfolge erfolgen, doch wird vorzugsweise die Lösung des Dialkalimetallsalzes vorgelegt und das Phenylchlorformiat zugegeben. Dabei erweist es sich als vorteilhaft, das Phenylchlorformiat langsam und kontinuierlich, d.h. dosiert, zuzugeben. Während der Zugabe ist der pH-Wert des Reaktionsgemisches durch Zugabe von Alkalilauge soweit wie möglich konstant zu halten. Um ein gutes Durchmischen während der Zugabe zu erreichen, wird zweckmässig gerührt oder anderweitig durchmischt. Zudem erfolgt das Zusammenbringen zweckmässigerweise bei etwa Raumtemperatur. Allerdings können sowohl die Lösung wie auch das Phenylchlorformiat gegebenenfalls vorgängig auf leicht erhöhte Temperatur von etwa 30-35°C erwärmt werden.

Was die relativen Mengen an Dialkalimetallsalz der meso-2,3-Bis(benzylamino) bernsteinsäure und Phenylchlorformiat anbelangt, so beträgt das Molverhältnis Dialkalimetallsalz : Phenylchlorformiat geeigneterweise etwa 1 : 1 bis etwa 1 : 4, vorzugsweise etwa 1 : 2 bis etwa 1 : 3.

Während der Umsetzung des Phenylchlorformiats mit dem Dialkalimetallsalz der meso-2,3-Bis(benzylamino)bernsteinsäure wird der pH-Wert des Reaktionsgemisches zweckmässigerweise im Bereich von etwa 8 bis etwa 14, vorzugsweise im Bereich von etwa 9 bis etwa 11, gehalten. Wie bereits erwähnt, wird zur Beibehaltung dieses pH-Bereiches wässrige Lithium-, Natron- oder Kalilauge zugegeben, und zwar gleichzeitig mit dem Zusammenbringen der Reaktionsteilnehmer. Die Konzentration der zugebenen Lithium-, Natron- oder Kalilauge ist nicht kritisch, beträgt allerdings geeigneterweise etwa 20 bis etwa 50 Gewichtsprozent. Besonders geeignet wird dazu die gleiche Lithium-, Natron- bzw. Kalilauge verwendet wie diejenige, welche zur Zubereitung des Dialkalimetallsalzes der meso-2,3-Bis(benzylamino)bernsteinsäure verwendet wurde.

Die Umsetzung erfolgt bei einer etwa 40°C nicht übersteigenden Temperatur, und zwar im allgemeinen bei Temperaturen im Bereich von etwa 25°C bis etwa 35°C, vorzugsweise um etwa 30°C. Der Druck ist nicht kritisch; es wird normalerweise unter Atmosphärendruck oder leicht erhöhtem Druck verfahren.

Die Durchführung des erfindungsgemässen Verfahrens erfolgt zweckmässig unter einer Inertgasatmosphäre. Im Falle der Verwendung einer Inertgasatmosphäre eignet sich als Inertgas insbesondere Stickstoff oder Argon, wobei im industriellen Massstab Stickstoff bevorzugt ist.

Nach erfolgter Zugabe, welche in der Regel etwa 2 bis 5 Stunden dauert, ist die Reaktion normalerweise auch beendet. Das resultierende einphasige Gemisch, welches als Produkt das Dialkalimetallsalz der 2-Oxo-1,3-dibenzyl-cis-4,5-imidazolidindicarbonsäure enthält, kann dann aufgearbeitet werden, indem das Gemisch durch Zugabe von Säure auf leicht alkalisch, insbesondere auf pH etwa 7-8, eingestellt und mit einem aromatischen Kohlenwasserstoff, vorzugsweise Toluol oder Xylol, extrahiert wird. Als Säure zum Ansäuern eignet sich insbesondere eine Mineralsäure, z.B. Salzsäure, Bromwasserstoffsäure oder Schwefelsäure, vorzugsweise Salzsäure. Das gewünschte Produkt, noch als Dialkalimetallsalz, befindet sich in der wässrigen Phase. Der getrennten wässrigen Phase wird dann geeigneterweise Tetrahydrofuran zugesetzt, und durch Ansäuern wird das Dialkalimetallsalz in die freie Säure übergeführt und als solche in die organische Phase aufgenommen. Als Säure zum Ansäuern wird zweckmässigerweise eine Mineralsäure verwendet, insbesondere Salzsäure, Bromwasserstoffsäure oder Schwefelsäure, vorzugsweise Salzsäure, deren jeweilige Konzentration und Menge zweckmässigerweise so gewählt werden, dass die wässrige Phase schliesslich einen pH-Wert von < 3, vorzugsweise um etwa 1, aufweist. Die das gewünschte Produkt als freie Säure enthaltende, von der wässrigen Phase abgetrennte organische Phase kann dann nach Zugabe eines aromatischen Kohlenwasserstoffes als Lösungsmittel, z.B. Toluol oder Xylol, einer Destillation unter vermindertem Druck unterworfen werden, um hauptsächlich das Tetrahydrofuran, wenig aromatisches Lösungsmittel sowie verbleibendes Wasser zu entfernen. In der resultierenden übersättigten Lösung des gewünschte Produktes in wasserfreiem aromatischem Lösungsmittel fällt dann, eventuell durch Animpfung und/oder unter Abkühlen induziert, die 2-Oxo-1,3-dibenzyl-cis-4,5-imidazolidindicarbonsäure als Kristalle aus.

Die so isolierte 2-Oxo-1,3-dibenzyl-cis-4,5-imidazolidindicarbonsäure kann in an sich bekannter Weise gewaschen, zweckmässigerweise mit Toluol, getrocknet und gewünschtenfalls weiter gereinigt werden.

Die einphasige Reaktionsführung und die Kristallisation aus einem aromatischen Kohlenwasserstoff bieten folgende Vorteile:
- Sichere Reaktionsführung ohne Ausfällungen von Diphenylcarbonat oder meso-2,3-Bis(benzylamino)bernsteinsäure
- Kein Verkleben der Reaktoreinbauten samt Rührer
- Genaue Kontrolle des pH-Wertes während der Reaktionsdauer
- Reduktion der Menge an Phenylchlorformiat im Vergleich zum Standard-Verfahren
- Reproduzierbare Fällung der 2-Oxo-1,3-dibenzyl-cis-4,5-imidazolidindicarbonsäure als feinkörniger Feststoff
- Verbesserte Ausbeute

Im Rahmen der vorliegenden Erfindung wurde festgestellt, dass es durch eine einfache Modifikation der Verfahrensführung gegen Ende der oben beschriebenen Aufarbeitung erstmals möglich ist, ohne Isolierung der 2-Oxo-1,3-dibenzyl-cis-4,5-imidazolidindicarbonsäure zum 2-Oxo-1,3-dibenzyl-4,5-imidazolidindicarbonsäureanhydrid, dem Produkt der nächsten Stufe in der Synthese von Biotin, zu gelangen.

Während man gegen Ende der oben beschriebenen Aufarbeitung, d.h. unmittelbar nach Abdestillation der organischen Lösungsmittel, die 2-Oxo-1,3-dibenzyl-cis-4,5-imidazolidindicarbonsäure auskristallisiert und zugleich isoliert, erfolgt bei der modifizierten Verfahrensführung die Zugabe von zusätzlichem aromatischen Lösungsmittel, vorzugsweise Toluol, sowie von Acetanhydrid. Zu diesem Zweck verwendet man geeigneterweise mindestens ein Mol Acetanhydrid pro Mol Dialkalimetallsalz der meso-2,3-Bis(benzylamino)bernsteinsäure, welches als Ausgangsmaterial verwendet wurde, vorzugsweise etwa 1,05 bis etwa 1,3 Mol Acetanhydrid pro Mol Ausgangsmaterial. Bei nachfolgendem Erhitzen auf etwa 70°C bis etwa 130°C, vorzugsweise etwa 75°C bis etwa 95°C, besonders bevorzugt etwa 80°C, reagiert die 2-Oxo-1,3-dibenzyl-cis-4,5-imidazolidindicarbonsäure gemäss untenstehendem Formelschema zu 2-Oxo-1,3-dibenzyl-4,5- imidazolidindicarbonsäureanhydrid, das aus der Lösung ausfällt und nach an sich bekannten Methoden, z.B. durch Filtration, isoliert werden kann.

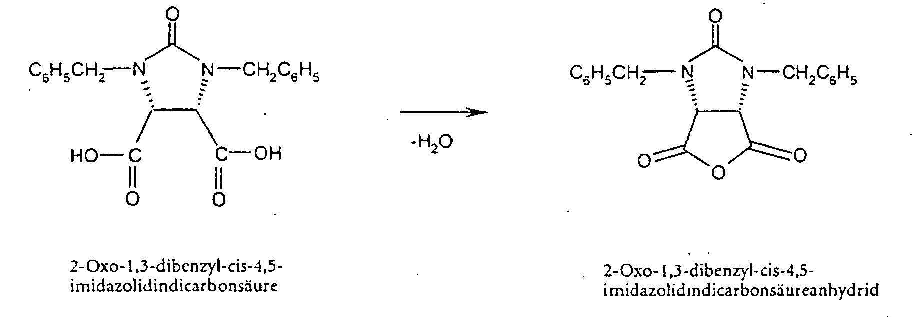

Somit beinhaltet die vorliegende Erfindung auch ein Verfahren zur Herstellung von 2-Oxo-1,3-dibenzyl-4,5-imidazolidindicarbonsäureanhydrid, wobei nach Durchführung des oben definierten Verfahrens zur Herstellung von 2-Oxo-1,3-dibenzyl-cis-4,5-imidazolidindicarbonsäure ausgehend von einem Dialkalimetallsalz der meso-2,3-Bis(benzylamino)bernsteinsäure und vor Isolierung der resultierenden 2-Oxo-1,3-dibenzyl-cis-4,5-imidazolidindicarbonsäure diese durch Erhitzen mit Acetanhydrid in einem aromatischen Kohlenwasserstoff als organischem Lösungsmittel, vorzugsweise Toluol, in das gewünschte 2-Oxo-1,3-dibenzyl-4,5-imidazolidindicarbonsäureanhydrid überführt und isoliert wird.

Die isolierte 2-Oxo-1,3-dibenzyl-cis-4,5-imidazolidindicarbonsäureanhydrid kann in an sich bekannter Weise gewaschen, zweckmässigerweise mit Toluol, getrocknet und gewünschtenfalls weiter gereinigt werden.

Die Umsetzung zum 2-Oxo-1,3-dibenzyl-4,5-imidazolidindicarbonsäureanhydrid in beispielsweise Toluol als Lösungsmittel verhält sich damit genauso, wie die Dehydratisierung von 2-Oxo-1,3-dibenzyl-4,5-imidazolidindicarbonsäure zum Anhydrid in einer Mischung aus Essigsäure und Acetanhydrid, eine Methode, welche bisher verwendet worden ist.

Die Vorteile der Verfahrensführung ohne Isolieren der 2-Oxo-1,3-dibenzyl-4,5-imidazolidindicarbonsäure sind:
- Keine Isolierung von 2-Oxo-1,3-dibenzyl-4,5-imidazolidindicarbonsäure benötigt
- Vollständige und einfache Kristallisation von 2-Oxo-1,3-dibenzyl-4,5-imidazolidindicarbonsäureanhydrid
- Sauberes Produkt
- Reduzierter apparativer und personeller Aufwand
- Ersatz von Essigsäure durch Toluol
- 2-Oxo-1,3-dibenzyl-4,5-imidazolidindicarbonsäure muss nicht getrocknet, abgefüllt und wieder eingefüllt werden
- Keine Mischungen von Essigsäure/Acetanhydrid einzustellen und zu destillieren
- Reduktion der Menge an Acetanhydrid um wenigstens 30 %
- Verbesserte Ausbeute verglichen mit der nach Abschluss der beiden Einzelstufen resultierenden Ausbeute

Das erfindungsgemässe Verfahren wird durch die nachfolgenden Beispiele veranschaulicht:

### Beispiel 1

In einem mit zwei Tropftrichtern, einer pH-Elektrode und einem Rührer versehenen 2 1-Kolben wurden unter Stickstoffatmosphäre 220 g (0,67 Mol) meso-2,3-Bis (benzylamino)bernsteinsäure vorgelegt. Die meso-2,3-Bis (benzylamino)bernsteinsäure wurde dann unter Rühren in 800 ml Tetrahydrofuran und 400 ml entionisiertem Wasser aufgeschlämmt und durch Zugabe von 108 ml 50%iger Kalilauge in Lösung gebracht. Zur erhaltenen Lösung wurden innerhalb von 3,5 Stunden 200 ml 2,4 Moläq Phenylchlorformiat zugetropft. Der pH-Wert wurde während der Zugabe von Phenylchlorformiat durch sukzessive Zugabe von 50%iger Kalilauge zwischen 9 und 11, und die Innentemperatur zwischen 30 und 35°C gehalten. Nach beendeter Zugabe wurde weitere 2 Stunden bei etwa 30°C gerührt und dann mit konzentrierter Salzsäure ein pH-Wert von etwa 7-8 eingestellt. Die wässrige Lösung wurde zweimal mit Toluol extrahiert. Zur gesamten wässrigen Phase wurden 500 ml Tetrahydrofuran gegeben. Dann wurde mit 37%iger Salzsäure ein pH-Wert von 1,0 eingestellt und die wässrige Phase abgetrennt. Der Tetrahydrofuran-Phase wurden 400 ml Toluol zugesetzt, und das Tetrahydrofuran wurde unter vermindertem Druck abdestilliert. Danach wurde noch verbliebenes Wasser azeotrop mit Toluol entfernt. Die zurückgebliebene bräunliche, ölige Lösung wurde angeimpft, der kristalline Feststoff abfiltriert und zweimal mit Toluol gewaschen. Nach Trocknen unter vermindertem Druck bis zur Gewichtskonstanz erhielt man 181,5 g (76%ige Ausbeute) 2-Oxo-1,3-dibenzyl-cis-4,5-imidazolidindicarbonsäure, Smp. 172,8°C.

### Beispiel 2

In einem mit zwei Tropftrichtern, einer pH-Elektrode und einem Rührer versehenen 2 1-Kolben wurden unter Stickstoffatmosphäre 220 g (0,67 Mol) meso-2,3-Bis (benzylamino)bernsteinsäure vorgelegt. Die meso-2,3-Bis (benzylamino)bernsteinsäure wurde dann unter Rühren in 800 ml Tetrahydrofuran und 400 ml entionisiertem Wasser aufgeschlämmt und durch Zugabe von 108 ml 50%iger Kalilauge in Lösung gebracht. Zur erhaltenen Lösung wurden innerhalb von 3,5 Stunden 200 ml 2,4 Moläq Phenylchlorformiat zugetropft. Der pH-Wert wurde während der Zugabe von Phenylchlor-formiat durch sukzessive Zugabe von 50%iger Kalilauge zwischen 9 und 11, und die Innentemperatur zwischen 30 und 35°C gehalten. Nach beendeter Zugabe wurde weitere 2 Stunden bei etwa 30°C gerührt und dann mit konzentrierter Salzsäure ein pH-Wert von etwa 7-8 eingestellt. Die wässrige Lösung wurde zweimal mit Toluol extrahiert. Zur gesamten wässrigen Phase wurden 500 ml Tetrahydrofuran gegeben. Dann wurde mit 37%iger Salzsäure ein pH-Wert von 1,0 eingestellt und die wässrige Phase abgetrennt. Der Tetrahydrofuran-Phase wurden 400 ml Toluol zugesetzt, und das Tetrahydrofuran wurde unter vermindertem Druck abdestilliert. Danach wurde noch verbliebenes Wasser azeotrop mit Toluol entfernt. Nach Zugabe von 700 ml Toluol und 130 ml Acetanhydrid wurde die Mischung auf 80°C erhitzt. Nachdem das 2-Oxo-1,3-dibenzyl-4,5-imidazolidindicarbonsäureanhydrid auskristallisiert war, wurde die erhaltene Suspension zur Vervollständigung der Kristallisation innerhalb von zwei Stunden auf 10°C abgekühlt und danach das kristalline 2-Oxo-1,3-dibenzyl-4,5-imidazolidindicarbonsäureanhydrid abfiltriert und zweimal mit Toluol gewaschen. Nach dem Trocknen unter vermindertem Druck bis zur Gewichtskonstanz erhielt man 173,6 g (78%ige Ausbeute) 2-Oxo-1,3-dibenzyl-4,5-imidazolidindicarbonsäureanhydrid in Form eines analysenreinen, farblosen Feststoffs, Smp. 240,2°C. Beim Eindampfen der Mutterlauge erhielt man nochmals 3,0 g Kristalle von 2-Oxo-1,3-dibenzyl-4,5-imidazolidindicarbonsäureanhydrid.

## Patentansprüche

1. Verfahren zur Herstellung von 2-Oxo-1,3-dibenzyl-cis-4,5-imidazolidindicarbonsäure bzw. 2-Oxo-1,3-dibenzyl-4,5-imidazolidindicarbonsäureanhydrid ausgehend von einem Dialkalimetallsalz der meso-2,3-Bis(benzylamino)bernsteinsäure, **dadurch gekennzeichnet, dass** man
das Dialkalimetallsalz der meso-2,3-Bis(benzylamino)bernsteinsäure mit Phenychlorformiat in einem aus einem etwa 2:1- bis 1:1-Gemisch von einem mit Wasser mischbaren Ether und wässriger Alkalilauge bestehenden Einphasen-Lösungsmittelsystem bei einer etwa 40°C nicht übersteigenden Temperatur umsetzt,
das daraus resultierende einphasige Gemisch, welches als Produkt das Dialkalimetallsalz der 2-Oxo-1,3-dibenzyl-cis-4,5-imidazolidindicarbonsäure enthält, durch Zugabe von Säure auf leicht alkalisch, insbesondere auf pH etwa 7-8, einstellt und mit einem aromatischen Kohlenwasserstoff extrahiert,
die resultierende wässrige Phase, in welcher sich das Produkt noch als Dialkalimetailsalz befindet, abtrennt,
der getrennten wässrigen Phase Tetrahydrofuran zusetzt,
das resultierende wässrig-organische Gemisch auf einen pH-Wert von < 3 (wässrige Phase) ansäuert, um das Dialkalimetallsalz in die freie Säure 2-Oxo-1,3-dibenzyl-cis-4,5-imidazolidindicarbonsäure überzuführen und letztere in die organische Phase aufzunehmen,
die organische Phase, welche die freie Säure enthält, von der wässrigen Phase abtrennt,
der organischen Phase einen aromatischen Kohlenwasserstoff als Lösungsmittel zugibt,
die so erhaltene organische Phase einer Destillation unter vermindertem Druck unterwirft, um hauptsächlich das Tetrahydrofuran, wenig aromatisches Lösungsmittel sowie verbleibendes Wasser zu entfernen,
und entweder aus der resultierenden übersättigten Lösung des gewünschten Produktes in wasserfreiem aromatischem Lösungsmittel die 2-Oxo-1,3-dibenzyl-cis-4,5-imidazolidindicarbonsäure fällt und diese isoliert
oder zu der resultierenden übersättigten Lösung zusätzlichen aromatischen Kohlenwasserstoff als organisches Lösungsmittel sowie Acetanhydrid zugibt und das Gemisch erhitzt, um die 2-Oxo-1,3-dibenzyl-cis-4,5-imidazolidindicarbonsäure in das alternativ gewünschte 2-Oxo-1,3-dibenzyl-4,5-imidazolidindicarbonsäureanhydrid überzuführen, und dieses isoliert.

2. Verfahren nach Anspruch 1, **dadurch gekennzeichnet, dass** man bei der Herstellung von 2-Oxo-1,3-dibenzylamino-4,5-imidazolidindicarbonsäureanhydrid als aromatischen Kohlenwasserstoff Toluol verwendet.

3. Verfahren nach Anspruch 1 oder 2, **dadurch gekennzeichnet, dass** man als mit Wasser mischbaren Ether Tetrahydrofuran oder einen Ethylenglycolether, z.B. Glyme oder Diglyme, vorzugsweise Tetrahydrofuran, verwendet.

4. Verfahren nach einem der Ansprüchel bis 3, **dadurch gekennzeichnet, dass** als Ausgangsmaterial das Dilithiumsalz, Dinatriumsalz oder Dikaliumsalz der meso-2,3-Bis(benzylamino)bernsteinsäure, vorzugsweise das letztgenannte, verwendet wird.

5. Verfahren nach einem der Ansprüche 1 bis 4, **dadurch gekennzeichnet, dass** zur Zubereitung des Dialkalimetallsalzes der meso-2,3-Bis(benzylamino)bernsteinsäure die Säure selber in Wasser aufgeschlämmt und die resultierende Aufschlämmung mit Alkalilauge, vorzugsweise Kalilauge, versetzt wird, um eine alkalische wässrige Lösung des erwünschten Dialkalimetallsalzes zu erhalten.

6. Verfahren nach einem der Ansprüche 1 bis 5, **dadurch gekennzeichnet, dass** man vor oder nach Zubereitung der wässrigen Dialkalimetallsalzlösung den mit Wasser mischbaren Ether zugibt.

7. Verfahren nach einem der Ansprüche 1 bis 6, **dadurch gekennzeichnet, dass** vor der Umsetzung die alkalisch-wässrige Ether-Lösung und/oder das Phenylchlorformiat auf eine erhöhte Temperatur von etwa 30-40°C erwärmt worden ist.

8. Verfahren nach einem der Ansprüche 1 bis 7, **dadurch gekennzeichnet, dass** das Molverhältnis Dialkalimetallsalz der meso-2,3-Bis(benzylamino)bernsteinsäure : Phenylchlorformiat etwa 1 : 1 bis etwa 1 : 4, vorzugsweise etwa 1 : 2 bis etwa 1 : 3, beträgt.

9. Verfahren nach einem der Ansprüche 1 bis 8, **dadurch gekennzeichnet, dass** während der Umsetzung des Dialkalimetallsalzes der meso-2,3-Bis(benzylamino) bernsteinsäure mit dem Phenylchlorformiat der pH-Wert des Reaktionsgemisches im Bereich von etwa 8 bis etwa 14, vorzugsweise im Bereich von etwa 9 bis etwa 11, gehalten wird.

10. Verfahren nach einem der Ansprüche 1 bis 9, **dadurch gekennzeichnet, dass** die Umsetzung bei einer Temperatur im Bereich von etwa 25°C bis etwa 35°C, vorzugsweise um etwa 30 °C, erfolgt.

11. Verfahren nach einem der Ansprüche 1 bis 10, **dadurch gekennzeichnet, dass** zum Ansäuern des erhaltenen Dialkalimetallsalzes der 2-Oxo-1,3-dibenzyl-cis-4,5-imidazolidindicarbonsäure eine Mineralsäure, insbesondere Salzsäure, Bromwasserstoffsäure oder Schwefelsäure, vorzugsweise Salzsäure, verwendet wird.

12. Verfahren nach einem der Ansprüche 1 bis 11, **dadurch gekennzeichnet, dass** man die erhaltene 2-Oxo-1,3-dibenzylamino-cis-4,5-imidazolidindicarbonsäure ohne Isolierung mit einem aromatischen Kohlenwasserstoff, vorzugsweise Toluol, und Acetanhydrid versetzt und das Reaktionsgemisch auf etwa 70°C bis etwa 130°C, vorzugsweise etwa 75°C bis etwa 95°C, besonders bevorzugt etwa 80°C, erhitzt.

13. Verfahren nach einem der Ansprüche 1 bis 12, **dadurch gekennzeichnet, dass** zur Isolierung der 2-Oxo-1,3-dibenzyl-cis-4,5-imidazolidindicarbonsäure bzw. des 2-Oxo-1,3-dibenzylamino-4,5-imidazolidindicarbonsäureanhydrids diese bzw. dieses aus einer Lösung in einem aromatischen Kohlenwasserstoff kristallisiert wird.

## Claims

1. A process for the production of 2-oxo-1,3-dibenzyl-4,5-imidazolidinedicarboxylic acid and, respectively, of 2-oxo-1,3-dibenzyl-4,5-imidazolidinedicarboxylic acid anhydride starting from a meso-2,3-bis(benzylamino)succinic acid dialkali metal salt, which process comprises reacting the meso-2,3-bis(benzylamino)succinic acid dialkali metal salt with phenyl chloroformate in a monophasic solvent system consisting of an about 2:1 to 1:1 mixture of a water-miscible ether and aqueous alkali metal hydroxide solution at a temperature not exceeding about 40°C, bringing the thus obtained monophasic mixture containing the 2-oxo-1,3-dibenzyl-cis-4,5-imidazolidinedicarboxylic acid dialkali metal salt by acidification to a slight alkaline pH, especially of about 7-8, and extracting it with an aromatic hydrocarbon,
separating the aqueous phase containing the product still as a dialkali metal salt,
adding to the separated aqueous phase tetrahydrofuran,
acidifying the resulting aqueous-organic mixture to a pH < 3 (aqueous phase) in order to convert the dialkali metal salt into the free 2-oxo-1,3-dibenzyl-cis-4,5-imidazolidinedicarboxylic acid and transferring the latter into the organic phase,
separating the organic phase containing the free acid from the aqueous phase,
adding to the organic phase an aromatic hydrocarbon as solvent,
distilling the thus obtained organic phase under reduced pressure in order to remove mainly the tetrahydrofuran, little aromatic solvent and residual water,
and either precipitating and isolating the 2-oxo-1,3-dibenzyl-cis-4,5-imidazolidinedicarboxylic acid from the supersaturated solution of the desired product in the anhydrous aromatic solvent or adding to the resulting supersaturated solution additional aromatic hydrocarbon as organic solvent as well as acetic anhydride and heating the mixture in order to convert the 2-oxo-1,3-dibenzyl-cis-4,5-imidazolidinedicarboxylic acid into the alternatively desired 2-oxo-1,3-dibenzyl-cis-4,5-imidazolidinedicarboxylic acid anhydride and isolating it.

2. A process according to claim 1, wherein toluene is used as the aromatic hydrocarbon in the production of 2-oxo-1,3-dibenzyl-cis-4,5-imidazolidinedicarboxylic acid anhydride.

3. A process according to claim 1 or 2, wherein tetrahydrofuran or an ethylene glycol ether, such as glyme or diglyme, preferably tetrahydrofuran, is used as the water-miscible ether.

4. A process according to any one of claims 1 to 3, wherein the dilithium salt, disodium salt or dipotassium salt of meso-2,3-bis(benzylamino)succinic acid, preferably the last-named, is used as the starting material.

5. A process according to any one of claims 1 to 4, wherein the meso-2,3-bis(benzylamino)succinic acid dialkali metal salt is produced by suspending the acid itself in water and treating the resulting suspension with alkali metal hydroxide solution, preferably potassium hydroxide solution, in order to obtain an alkaline-aqueous solution of the desired dialkali metal salt.

6. A process according to any one of claims 1 to 5, wherein the water-miscible ether is added before or after the production of the aqueous dialkali metal salt solution.

7. A process according to any one of claims 1 to 6, wherein the alkaline-aqueous ether solution and/or the phenyl chloroformate is/are warmed to an elevated temperature of about 30-40°C before the reaction.

8. A process according to any one of claims 1 to 7, wherein the meso-2,3-bis(benzylamino)succinic acid dialkali metal salt : phenyl chloroformate molar ratio is about 1 : 1 to about 1 : 4, preferably about 1 : 2 to about 1 : 3.

9. A process according to any one of claims 1 to 8, wherein the pH value of the reaction mixture is held in the range of about 8 to about 14, preferably in the range of about 9 to about 11, during the reaction of the meso-2,3-bis(benzylamino)succmic acid dialkali metal salt with the phenyl chloroformate.

10. A process according to any one of claims 1 to 9, wherein the reaction is carried out at a temperature in the range of about 25°C to about 35°C, preferably at about 30°C.

11. A process according to any one of claims 1 to 10, wherein a mineral acid, especially hydrochloric acid, hydrobromic acid or sulphuric acid, preferably hydrochloric acid, is used for the acidification of the 2-oxo-1,3-dibenzyl-cis-4,5-imidazolidinedicarboxylic dialkali metal salt obtained.

12. A process according to any one of claims 1 to 11, wherein the 2-oxo-1,3-dibenzyl-cis-4,5-imidazolidinedicarboxylic acid obtained is treated without isolation with an aromatic hydrocarbon, preferably toluene, and acetic anhydride and the reaction mixture is heated to about 70°C to about 130°C, preferably about 75°C to about 95°C, especially about 80°C.

13. A process according to any one of claims 1 to 12, wherein the 2-oxo-1,3-dibenzyl-4,5-imidazolidinedicarboxylic acid or the 2-oxo-1,3-dibenzyl-4,5-imidazolidinedicarboxylic acid anhydride is isolated by crystallization from a solution in an aromatic hydrocarbon.

## Revendications

1. Procédé pour la préparation d'acide 2-oxo-1,3-dibenzyl-*cis*-4,5-imidazolidinedicarboxylique ou d'anhydride d'acide 2-oxo-1,3-dibenzyl-4,5-imidazolidinedicarboxylique à partir d'un disel de métal alcalin de l'acide méso-2,3-bis(benzylamino)succinique, **caractérisé en ce qu'**on
fait réagir le disel de métal alcalin de l'acide méso-2,3-bis(benzylamino)-succinique avec du chloroformiate de phényle dans un système de solvant monophasique consistant en un mélange d'environ 2:1 à 1:1 d'un éther miscible à l'eau et d'une solution aqueuse d'un hydroxyde de métal alcalin, à une température n'excédant pas environ 40°C,
ajuste à un pH légèrement alcalin, en particulier à pH environ 7-8, le mélange monophasique résultant qui contient en tant que produit le disel de métal alcalin de l'acide 2-oxo-1,3-dibenzyl-*cis*-4,5-imidazolidinedicarboxylique, par addition d'un acide, et on l'extrait à l'aide d'un hydrocarbure aromatique,
sépare la phase aqueuse résultante, dans laquelle se trouve le produit encore sous forme de disel de métal alcalin,
ajoute du tétrahydrofuranne à la phase aqueuse séparée,
acidifie à un pH < 3 (phase aqueuse) le mélange aqueux-organique résultant, pour transformer le disel de métal alcalin en l'acide 2-oxo-1,3-dibenzyl-*cis*-4,5-imidazolidinedicarboxylique libre et pour reprendre ce dernier dans la phase organique,
sépare la phase organique, qui contient l'acide libre, d'avec la phase aqueuse,
ajoute à la phase organique un hydrocarbure aromatique en tant que solvant,
soumet à une distillation sous pression réduite la phase organique ainsi obtenue, pour éliminer principalement le tétrahydrofuranne, un peu de solvant aromatique ainsi que l'eau restante,
et soit on fait précipiter l'acide 2-oxo-1,3-dibenzyl-*cis*-4,5-imidazolidinedicarboxylique à partir de la solution sursaturée résultante du produit recherché, dans un solvant aromatique anhydre, et on l'isole,
soit on ajoute de l'hydrocarbure aromatique supplémentaire en tant que solvant organique ainsi que de l'anhydride acétique à la solution sursaturée résultante et on chauffe le mélange, afin de transformer l'acide 2-oxo-1,3-dibenzyl-*cis*-4,5-imidazolidinedicarboxylique en l'anhydride d'acide 2-oxo-1,3-dibenzyl-4,5-imidazolidinedicarboxylique également recherché, et on l'isole.

2. Procédé selon la revendication 1, **caractérisé en ce que**, dans la préparation d'anhydride d'acide 2-oxo-1,3-dibenzylamino-4,5-imidazolidinedicarboxylique, on utilise du toluène en tant qu'hydrocarbure aromatique.

3. Procédé selon la revendication 1 ou 2, **caractérisé en ce qu'**on utilise en tant qu'éther miscible à l'eau du tétrahydrofuranne ou un éther d'éthylèneglycol, par exemple le glyme ou le diglyme, de préférence le tétrahydrofuranne.

4. Procédé selon l'une quelconque des revendications 1 à 3, **caractérisé en ce qu'**on utilise en tant que produit initial le disel de lithium, le disel de sodium ou le disel de potassium de l'acide méso-2,3-bis(benzylamino)succinique, de préférence celui cité en dernier.

5. Procédé selon l'une quelconque des revendications 1 à 4, **caractérisé en ce que**, pour la préparation du disel de métal alcalin de l'acide méso-2,3-bis(benzylamino)succinique, on met l'acide lui-même en suspension dans l'eau et on traite la suspension résultante avec une solution d'un hydroxyde alcalin, de préférence une solution d'hydroxyde de potassium, pour obtenir une solution aqueuse alcaline du disel de métal alcalin désiré.

6. Procédé selon l'une quelconque des revendications 1 à 5, **caractérisé en ce qu'**on ajoute l'éther miscible à l'eau avant ou après la préparation de la solution aqueuse de disel de métal alcalin.

7. Procédé selon l'une quelconque des revendications 1 à 6, **caractérisé en ce que** la solution aqueuse-alcaline d'éther et/ou le chloroformiate de phényle a(ont) été chauffé(e)(s) à une température élevée d'environ 30-40°C, avant la réaction.

8. Procédé selon l'une quelconque des revendications 1 à 7, **caractérisé en ce que** le rapport molaire du disel de métal alcalin de l'acide méso-2,3-bis(benzylamino)succinique:chloroformiate de phényle va d'environ 1:1 à environ 1:4, de préférence d'environ 1:2 à environ 1:3.

9. Procédé selon l'une quelconque des revendications 1 à 8, **caractérisé en ce que** pendant la réaction du disel de métal alcalin de l'acide méso-2,3-bis(benzylamino)succinique avec le chloroformiate de phényle, on maintient le pH du mélange réactionnel dans l'intervalle allant d'environ 8 à environ 14, de préférence dans l'intervalle allant d'environ 9 à environ 11.

10. Procédé selon l'une quelconque des revendications 1 à 9, **caractérisé en ce que** la réaction s'effectue à une température dans la plage d'environ 25°C à environ 35°C, de préférence à une température d'environ 30°C.

11. Procédé selon l'une quelconque des revendications 1 à 10, **caractérisé en ce que**, pour l'acidification du disel de métal alcalin obtenu de l'acide 2-oxo-1,3-dibenzyl-*cis*-4,5-imidazolidinedicarboxylique, on utilise un acide minéral, en particulier de l'acide chlorhydrique, de l'acide bromhydrique ou de l'acide sulfurique, de préférence de l'acide chlorhydrique.

12. Procédé selon l'une quelconque des revendications 1 à 11, **caractérisé en ce que** l'on traite l'acide 2-oxo-1,3-dibenzylamino-cis-4,5-imidazolidinedicarboxylique obtenu sans isolement avec un hydrocarbure aromatique, de préférence du toluène, et on traite avec de l'anhydride acétique et on chauffe le mélange réactionnel à environ 70°C jusqu'à environ 130°C, de préférence environ 75°C à environ 95°C, de manière particulièrement préférée environ 80°C.

13. Procédé selon l'une quelconque des revendications 1 à 12, **caractérisé en ce que** pour l'isolement de l'acide 2-oxo-1,3-dibenzyl-cis-4,5-imidazolidinedicarboxylique ou de l'anhydride d'acide 2-oxo-1,3-dibenzylamino-4,5-imidazolidinedicarboxylique on cristallise celui-ci ou celui-là d'une solution d'un hydrocarbure aromatique.
